(19) Europäisches Patentamt
European Patent Office
Office européen des brevets

(11) EP 1 020 188 A1

(12) # EUROPEAN PATENT APPLICATION

(43) Date of publication:
**19.07.2000 Bulletin 2000/29**

(51) Int Cl.[7]: **A61K 31/335**

(21) Application number: **99400072.7**

(22) Date of filing: **13.01.1999**

(84) Designated Contracting States:
**AT BE CH CY DE DK ES FI FR GB GR IE IT LI LU MC NL PT SE**
Designated Extension States:
**AL LT LV MK RO SI**

(71) Applicant: **Aventis Pharma S.A.**
**92165 Antony Cédex (FR)**

(72) Inventor: **Bissery, Marie-Christine**
**94400 Vitry sur Seine (FR)**

(74) Representative: **Le Pennec, Magali et al**
**RHONE-POULENC RORER SA,**
**Direction des Brevets,**
**20 Avenue Raymond Aron**
**92165 Antony Cédex (FR)**

(54) **New use of taxoid derivatives**

(57) The present invention relates a new pharmaceutical form of taxoid derivatives. It relates more precisely an oral administration of a taxoïd bearing two alkoxy groups in position 7 and 10.

EP 1 020 188 A1

## Description

The present invention relates a new pharmaceutical form of taxoid derivatives. It relates more precisely an oral administration of a compound of general formula (I) or a pharmaceutically acceptable salt or solvante thereof:

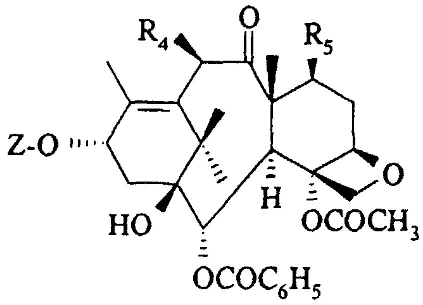

(I)

in which :

Z represents a hydrogen atom or a radical of general formula :

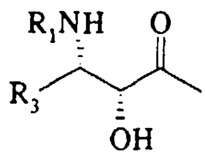

(II)

in which :

$R_1$ represents

a benzoyl radical optionally substituted with one or more identical or different atoms or radicals chosen from halogen atoms and alkyl radicals containing 1 to 4 carbon atoms, alkoxy radicals containing 1 to 4 carbon atoms or trifluoromethyl radicals,

a thenoyl or furoyl radical or

a radical $R_2$-O-CO- in which $R_2$ represents

- an alkyl radical containing 1 to 8 carbon atoms,
- an alkenyl radical containing 2 to 8 carbon atoms,
- an alkynyl radical containing 3 to 8 carbon atoms,
- a cycloalkyl radical containing 3 to 6 carbon atoms,
- a cycloalkenyl radical containing 4 to 6 carbon atoms or
- a bicycloalkyl radical containing 7 to 10 carbon atoms, these radicals being optionally substituted with one or more substituents chosen from halogen atoms and hydroxyl radicals, alkoxy radicals containing 1 to 4 carbon atoms, dialkylamino radicals in which each alkyl portion contains 1 to 4 carbon atoms, piperidino or morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, phenyl radicals (optionally substituted with one or more atoms or radicals chosen from halogen atoms and alkyl radicals containing 1 to 4 carbon atoms or alkoxy radicals containing 1 to 4 carbon atoms), cyano or carboxyl radicals or alkoxycarbonyl radicals in which the alkyl portion contains 1 to 4 carbon atoms,
- a phenyl or α- or β-naphthyl radical optionally substituted with one or more atoms or radicals chosen from halogen atoms and alkyl radicals containing 1 to 4 carbon atoms or alkoxy radicals containing 1 to 4 carbon atoms, or
- a 5-membered aromatic heterocyclic radical preferably chosen from furyl and thienyl radicals,
- or a saturated heterocyclic radical containing 4 to 6 carbon atoms, optionally substituted with one or more alkyl radicals containing 1 to 4 carbon atoms,

$R_3$ represents

an unbranched or branched alkyl radical containing 1 to 8 carbon atoms,
an unbranched or branched alkenyl radical containing 2 to 8 carbon atoms,
an unbranched or branched alkynyl radical containing 2 to 8 carbon atoms,
a cycloalkyl radical containing 3 to 6 carbon atoms,
a phenyl or α- or β-naphthyl radical optionally substituted with one or more atoms or radicals chosen from halogen atoms and alkyl, alkenyl, alkynyl, aryl, aralkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro and trifluoromethyl radicals,
or a 5-membered aromatic heterocycle containing one or more identical or different hetero atoms chosen from nitrogen, oxygen and sulphur atoms and optionally substituted with one or more identical or different substituents chosen from halogen atoms and alkyl, aryl, amino, alkylamino, dialkylamino, alkoxycarbonylamino, acyl, arylcarbonyl, cyano, carboxyl, carbamoyl, alkylcarbamoyl, dialkylcarbamoyl or alkoxycarbonyl radicals,
on the understanding that, in the substituents of the phenyl, α- or β-naphthyl and aromatic heterocyclic radicals, the alkyl radicals and the alkyl portions of the other radicals contain 1 to 4 carbon atoms, and that the alkenyl and alkynyl radicals contain 2 to 8 carbon atoms, and that the aryl radicals are phenyl or α- or β-naphthyl radicals,
$R_4$ represents
an alkoxy radical containing 1 to 6 carbon atoms in an unbranched or branched chain,
an alkenyloxy radical containing 3 to 6 carbon atoms in an unbranched or branched chain,
an alkynyloxy radical containing 3 to 6 carbon atoms in an unbranched or branched chain,
a cycloalkyloxy radical containing 3 to 6 carbon atoms or
a cycloalkenyloxy radical containing 4 to 6 carbon atoms,
these radicals being optionally substituted with one or more halogen atoms or with an alkoxy radical containing 1 to 4 carbon atoms, an alkylthio radical containing 1 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur or nitrogen atoms, optionally substituted with an alkyl radical containing 1 to 4 carbon atoms or a phenyl radical or a phenylalkyl radical in which the alkyl portion contains 1 to 4 carbon atoms,
$R_5$ represents
an alkoxy radical containing 1 to 6 carbon atoms in an unbranched or branched chain,
an alkenyloxy radical containing 3 to 6 carbon atoms,
an alkynyloxy radical containing 3 to 6 carbon atoms,
a cycloalkyloxy radical containing 3 to 6 carbon atoms or
a cycloalkenyloxy radical containing 3 to 6 carbon atoms,
these radicals being optionally substituted with one or more halogen atoms or with an alkoxy radical containing 1 to 4 carbon atoms, an alkylthio radical containing 2 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur or nitrogen atoms, optionally substituted with an alkyl radical containing 1 to 4 carbon atoms or a phenyl radical or a phenylalkyl radical in which the alkyl portion contains 1 to 4 carbon atoms.

Preferably, the aryl radicals which can be represented by $R_3$ are phenyl or α-or β-naphthyl radicals optionally substituted with one or more atoms or radicals chosen from halogen atoms (fluorine, chlorine, bromine, iodine) and alkyl, alkenyl, alkynyl, aryl, arylalkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, dialkylcarbamoyl, cyano, nitro and trifluoromethyl radicals, on the understanding that the alkyl radicals and the alkyl portions of the other radicals contain 1 to 4 carbon atoms, that the alkenyl and alkynyl radicals contain 2 to 8 carbon atoms and that the aryl radicals are phenyl or α- or β-naphthyl radicals.

Preferably, the heterocyclic radicals which can be represented by $R_3$ are 5-membered aromatic heterocyclic radicals containing one or more identical or different atoms chosen from nitrogen, oxygen and sulphur atoms, optionally substituted with one or more identical or different substituents chosen from halogen atoms (fluorine, chlorine, bromine, iodine) and alkyl radicals containing 1 to 4 carbon atoms, aryl radicals containing 6 to 10 carbon atoms, alkoxy radicals containing 1 to 4 carbon atoms, aryloxy radicals containing 6 to 10 carbon atoms, amino radicals, alkylamino radicals containing 1 to 4 carbon atoms, dialkylamino radicals in which each alkyl portion contains 1 to 4 carbon atoms, acylamino radicals in which the acyl portion contains 1 to 4 carbon atoms, alkoxycarbonylamino radicals containing 1 to 4 carbon atoms, acyl radicals containing 1 to 4 carbon atoms, arylcarbonyl radicals in which the aryl portion contains 6 to 10 carbon atoms, cyano, carboxyl or carbamoyl radicals, alkylcarbamoyl radicals in which the alkyl portion contains

1 to 4 carbon atoms, dialkylcarbamoyl radicals in which each alkyl portion contains 1 to 4 carbon atoms or alkoxycarbonyl radicals in which the alkoxy portion contains 1 to 4 carbon atoms.

Preferably, the radicals $R_4$ and $R_5$, which may be identical or different, represent unbranched or branched alkoxy radicals containing 1 to 6 carbon atoms, optionally substituted with a methoxy, ethoxy, ethylthio, carboxyl, methoxycarbonyl, ethoxycarbonyl, cyano, carbamoyl, N-methylcarbamoyl, N-ethylcarbamoyl, N,N-dimethylcarbamoyl, N, N-diethylcarbamoyl, N-pyrrolidinocarbonyl or N-piperidinocarbonyl radical.

More specially, the present invention relates to the products of general formula (I) in which Z represents a hydrogen atom or a radical of general formula (II) in which $R_1$ represents a benzoyl radical or a radical $R_2$-O-CO- in which $R_2$ represents a tert-butyl radical and $R_3$ represents an alkyl radical containing 1 to 6 carbon atoms, an alkenyl radical containing 2 to 6 carbon atoms, a cycloalkyl radical containing 3 to 6 carbon atoms, a phenyl radical optionally substituted with one or more identical or different atoms or radicals chosen from halogen atoms (fluorine, chlorine) and alkyl (methyl), alkoxy (methoxy), dialkylamino (dimethylamino), acylamino (acetylamino), alkoxycarbonylamino (tert-butoxycarbonylamino) or trifluoromethyl radicals, or a 2- or 3-furyl, 2- or 3-thienyl or 2-, 4- or 5-thiazolyl radical, and $R_4$ and $R_5$, which may be identical or different, each represent an unbranched or branched alkoxy radical containing 1 to 6 carbon atoms.

Still more specially, the present invention relates to the products of general formula (I) in which Z represents a hydrogen atom or a radical of general formula (II) in which $R_1$ represents a benzoyl radical or a radical $R_2$-O-CO- in which $R_2$ represents a tert-butyl radical and $R_3$ represents an isobutyl, isobutenyl, butenyl, cyclohexyl, phenyl, 2-furyl, 3-furyl, 2-thienyl, 3-thienyl, 2-thiazolyl, 4-thiazolyl or 5-thiazolyl radical, and $R_4$ and $R_5$, which may be identical or different, each represent a methoxy, ethoxy or propoxy radical.

Of even more special interest are the products of general formula (I) in which $R_3$ represents a phenyl radical and $R_1$ represents a ter-butoxycarbonyl radical, $R_4$ and $R_5$, which may be identical or different, represent a methoxy, ethoxy or propoxy radical.

In a still higher interest, the present invention relates to 4α-acetoxy-2α-benzoyloxy-5β,20-epoxy-1β-hydroxy-7β, 10β-dimethoxy-9-oxo-11-taxen-13α-yl (2R,3S)-3 tert-butoxycarbonylamino-2-hydroxy-3-phenylpropionate of formula (Ia)

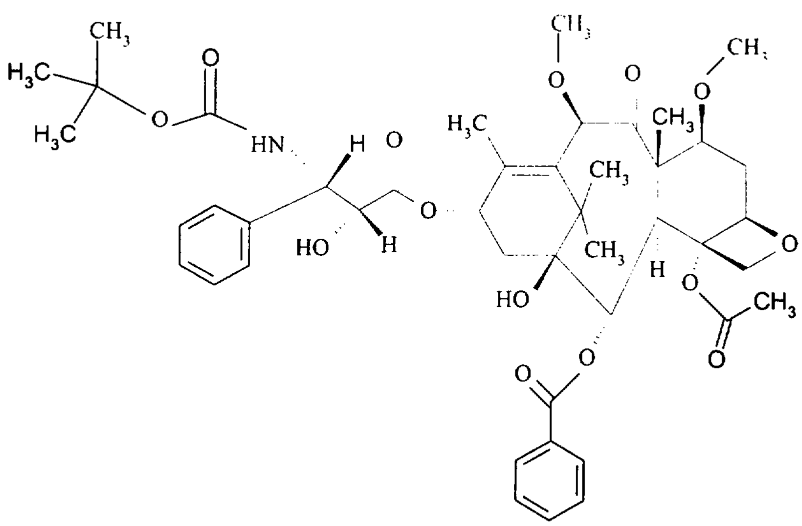

It is known, from patent WO 96/30355, to prepare a derivative according to the present invention by two processes. According to a first, multi-step process, starting with 10-deacetylbaccatin III of formula :

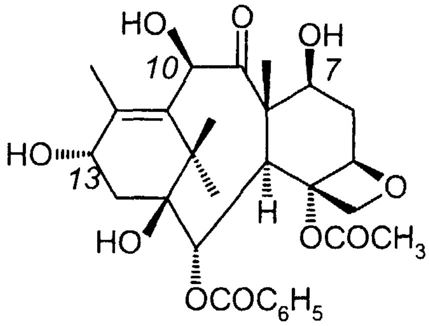

(III)

it is selectively protected in positions 7 and 13, for example in the form of a silyl diether, followed by the action of a product of general formula:

R-X (IV)

in which R represents a radical as defined above and X represents a reactive ester residue such as a sulphuric or sulphonic ester residue or a halogen atom, to give a product bearing the unit -OR in position 10 and silyl groups in positions 7 and 13. Next, the silyl protecting groups are replaced with hydrogen atoms to give a compound still bearing the group -OR in position 10 and OH groups in positions 7 and 13. The latter derivative is etherified selectively in position 7 by reaction with the derivative of formula IV to give the derivative of formula (I) in which Z is equal to hydrogen.

The final step consists in esterifying in position 13, according to a process which is known per se, the derivatives of formula (Ia), in which Z represents hydrogen, in the presence of a β-lactam according, for example, to the process described in patent EP 617,018, or in the presence of an oxazolidine as described, for example, in patent WO 96/30355 mentioned above. After deprotection of the protecting groups in positions 7 and 10, an ester of formula (Ia) is thus obtained in which Z is other than hydrogen and R represents hydrogen. The next step consists in reacting the positions 7 and 10 simultaneously by the action of a reagent formed in situ from a sulphoxide of formula (V) and acetic anhydride (Pummerer-type reaction),

R-SO-R (V)

in which R has the same meaning as above, to form an alkylthioalkyloxy-type intermediate on positions 7 and 10.

The final step, which allows the desired compound of formula (Ia) to be obtained, is carried out on the intermediate compound obtained above, by the action of activated Raney nickel.

Generally, the action of the reagent formed in situ from sulphoxide of general formula (V), preferably dimethyl sulphoxide and acetic anhydride, is carried out in the presence of acetic acid or an acetic acid derivative such as a haloacetic acid, at a temperature of between 0 and 50°C.

Generally, the action of the activated Raney nickel in the presence of an aliphatic alcohol or an ether is carried out at a temperature of between -10 and 60°C.

In the FR 97-14442 application a further process has been described. This invention allows, in a single step, the direct, selective and simultaneous alkylation of the two hydroxyl functions in positions 7 and 10 of 10-deacetylbaccatin or of derivatives thereof esterified in position 13, of formula (VI)

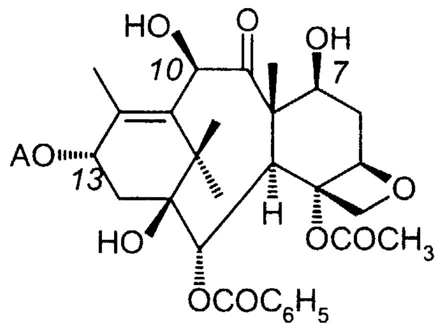

(VI)

in which A represents hydrogen or a side chain of formula (IIa) below :

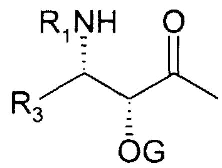

(IIa)

in which G represents a protecting group for the hydroxyl function, $R_1$ and $R_3$ have the same meaning as in formula (II) or an oxazolidine unit of formula (Id) :

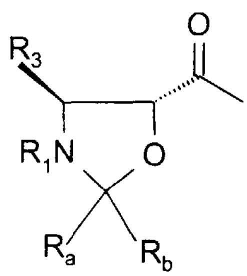

(IIb)

in which $R_1$ and $R_3$ have the same meaning as in formula (II), $R_a$ and $R_b$, which may be identical or different, represent hydrogen or alkyl, aryl, halo, alkoxy, arylalkyl, alkoxyaryl, haloalkyl, haloaryl, it being possible for the substituents optionally to form a 4- to 7-membered ring.

It is preferred to use 10-deacetylbaccatin as starting material, i.e. the product of formula (III), which allows appreciable economy as regards the process and moreover avoids the intermediate protection and deprotection steps necessary in the old processes.

Among the groups G for protecting the hydroxyl function of formula (IIa), it is generally preferred to choose all of the protecting groups described in books such as Greene and Wuts, Protective Groups in Organic Synthesis, 1991, John Wiley & Sons, and MacOmie, Protective Groups in Organic Chemistry, 1975, Plenum Press, and which are deprotected under conditions which degrade the rest of the molecule little or not at all, such as, for example:

- ethers, and preferably ethers such as methoxymethyl ether, 1-ethoxyethyl ether, benzyloxymethyl ether, p-methoxybenzyloxymethyl ether, benzyl ethers optionally substituted with one or more groups such as methoxy, chloro, nitro, 1-methyl-1-methoxyethyl ether, 2-(trimethylsilyl)ethoxymethyl ether, tetrahydropyranyl ether and silyl ethers such as trialkylsilyl ethers,
- carbonates such as trichloroethyl carbonates.

More particularly, the radicals $R_a$ and $R_b$ of general formula (IIb) are chosen from those described in patent WO 94/07878 and the derivatives more particularly preferred are those in which $R_a$ is hydrogen and $R_b$ is a p-methoxyphenyl radical.

The alkylating agent is chosen from :

- alkyl halides, and preferably from alkyl iodides (RI)
- alkyl sulphates such as methyl sulphate
- oxoniums such as trialkyloxonium boric salts, in particular trimethyloxonium tetrafluoroborate ($Me_3OBF_4$).

Methyl iodide is preferably used.

The alkylating agent is used in the presence of an anionization agent such as one or more strong bases, in anhydrous medium.

Among the bases which can be used in anhydrous medium, mention may be made of:

- alkali metal hydrides such as sodium or potassium hydride
- alkali metal alkoxides such as potassium tert-butoxide
- silver oxide $Ag_2O$
- 1,8-bis(dimethylamino)naphthalene
- mono- or dimetallic base mixtures such as those described, for example, in publications such as P. Caubère Chem. Rev. 1993, 93, 2317-2334 or M. Schlosser Mod. Synth. Methods (1992), 6, 227-271; in particular the alkyllithium/alkali metal t-butoxide or alkali metal amide/alkali metal t-butoxide combinations are preferred. One of the two bases can be generated "in situ".

Among all of the possible combinations of alkylating agent and anionization agent, it is preferred to use methyl iodide in the presence of potassium hydride.

The reaction is preferably carried out in an organic medium which is inert under the reaction conditions. Among the solvents, it is preferred to use :

- ethers such as tetrahydrofuran or dimethoxyethane
- when silver oxide is used, it is preferred to use polar aprotic solvents such as dimethylformamide, or aromatic solvents such as toluene
- when 1,8-bis(dimethylamino)naphthalene is used, it is preferred to use alkylesters such as ethylacetate.

For better implementation of the invention, it is preferred to use a molar ratio between the anionization agent and the substrate of greater than 2 and preferably between 2 and 20.

It is also preferred to use a molar ratio between the alkylating agent and the substrate of greater than 2 and preferably between 2 and 40.

It is preferred to use a reaction temperature of between -30°C and 80°C.

The reaction time advantageously ranges between a few hours and 48 hours depending on the reagents chosen.

After the alkylating step, when the latter is carried out on 10-deacetylbaccatin, the process then proceeds, in a known manner, to the esterification step according, for example, to the processes described in patents EP 617,018 or WO 96/30355 mentioned above.

Thus, according to a first, 3-step process, the procedure first begins with the dialkylation of 10-deacetylbaccatin, using an alkylating agent in the presence of a strong base, in a second step, the 10-deacetylbaccatin dietherified in positions 7 and 10 is coupled, in position 13, with a suitably protected β-lactam in the presence of an activating agent chosen from tertiary amines and metal bases which ensure the formation of an alkoxide in position 13. Deprotection of the side chain is then achieved by the action of an inorganic or organic acid.

Thus, according to a second, 3-step process, the procedure first begins with the dialkylation of 10-deacetylbaccatin, using an alkylating agent in the presence of a strong base, in a second step, the 10-deacetylbaccatin dietherified in positions 7 and 10 is coupled, in position 13, with an oxazolidine in the presence of a coupling agent such as diimides in the presence of an activating agent such as dialkylaminopyridines. Opening of the oxazolidine is achieved by the action of an inorganic or organic acid.

According to a third process, the procedure begins with the esterification in position 13 of baccatin suitably protected in positions 7 and 10, with a β-lactam or an oxazolidine in the presence of a coupling agent and/or an activating agent as described in the above two processes. After deprotection in positions 7 and 10, the dietherification in positions 7 and 10 is carried out by an alkylating agent in the presence of a strong base. Deprotection of the side chain is then achieved by the action of an inorganic or organic acid.

Formulations suitable for oral administration means formulations which are in a form suitable to be administered orally to a patient. The formulations may be presented as any suitable oral dosage form such as for example capsules, cachets or tablets each containing a predetermined amount of the active ingredient; as a powder or granules; as solution or a suspension in an aqueous liquid or a non-aqueous liquid; or as an oil-in-water liquid emulsion or a water-in-oil liquid emulsion. The active ingredient may also be presented as a bolus, electuary or paste.

Pharmaceutical composition means a composition comprising a compound of formula I and at least one component selected from the group comprising pharmaceutically acceptable carriers, diluents, adjuvants, excipients, or vehicles, such as preserving agents, fillers, disintegrating agents, wetting agents, emulsifying agents, suspending agents, sweetening agents, flavoring agents, perfuming agents, antibacterial agents, antifungal agents, lubricating agents and dispensing agents, depending on the nature of the mode of administration and dosage forms. Examples of suspending agents include ethoxylated isostearyl alcohols, polyoxyethylene sorbitol and sorbitan esters, microcrystalline cellulose, aluminum metahydroxide, bentonite, agar-agar and tragacanth, or mixtures of these substances. Prevention of the action of microorganisms can be ensured by various antibacterial and antifungal agents, for example, parabens, chlorobutanol, phenol, sorbic acid, and the like. Examples of suitable carriers, diluents, solvents or vehicles include water, ethanol, polyols, suitable mixtures thereof, vegetable oils (such as olive oil) and organic esters such as ethyl oleate. Examples of excipients include lactose, milk sugar, sodium citrate, calcium carbonate, dicalcium phosphate. Examples of disintegrating agents include starch, alginic acids and certain complex silicates. Examples of lubricants include magnesium stearate, sodium lauryl sulphate, talc, as well as high molecular weight polyethylene glycols.

Liquid dosage form means the dose of the active compound to be administered to the patient is in liquid form, for example, pharmaceutically acceptable emulsions, solutions, suspensions, syrups and elixirs. In addition to the active compounds, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils, in particular, cottonseed oil, groundnut oil, corn germ oil, olive oil, castor oil and sesame oil, glycerol, tetrahydrofurfuryl alcohol, polyethylene glycols, fatty acid esters of sorbitan and phospholipids or mixtures of these substances, and the like.

Solid dosage form means the dosage form of the compound useful according to the invention is solid form, for example capsules, tablets, pills, powders, dragees or granules. In such solid dosage forms, the compound useful according to the invention is admixed with at least one inert customary excipient (or carrier) such as sodium citrate or dicalcium phosphate or (a) fillers or extenders, as for example, starches, lactose, sucrose, glucose, mannitol and silicic acid, (b) binders, as for example, carboxymethylcellulose, alignates, gelatin, polyvinylpyrrolidone, sucrose and acacia, (c) humectants, as for example, glycerol, (d) disintegrating agents, as for example, agar-agar, calcium carbonate, potato or tapioca starch, alginic acid, certain complex silicates and sodium carbonate, (e) solution retarders, as for example paraffin, (f) absorption accelerators, as for example, quaternary ammonium compounds, (g) wetting agents, as for example, cetyl alcohol and glycerol monostearate, (h) adsorbents, as for example, kaolin and bentonite, (i) lubricants, as for example, talc, calcium stearate, magnesium stearate, solid polyethylene glycols, sodium lauryl sulfate, (j) opacifying agents, (k) buffering agents , and agents which release the compound(s) useful according to the invention in a certain part of the intestinal tract in a delayed manner.

The invention will be more accurately defined by the following examples which must not be considered as limitating the present invention.

Product of formula (Ia) is formulated as following :

Formulation II : 0,2 mg/ml of product of formula Ia in a solution containing 5 % ethanol, 5 % Polysorbate 80 and 90 % of an aqueous 5 % glucose solution. 15 female C3H/HeN mice each weighing ca 20 g were each administered orally with formulated product II to give a dose of 20.6 $mg.kg^{-1}$ per administration per mouse, 12,8 ; 8,4 and 5,5 each day for 5 days.

Animals : The animals subjected to the experiment, generally mice, are subcutaneously grafted bilaterally with 30 to 60 mg of a mammary tumor fragment (adenocarcinoma MA17/A) on day 0. The animals bearing tumors are mixed before being assigned to the various treatments and controls groups. Chemotherapy generally begins from 3 to 7 days after grafting, depending on the type of tumour, and the animals are observed every day. The different animal groups are weighed 3 or 4 times a week until the maximum weight loss is attained, and the groups are then weighed at least once a week until the end of the trial.

Efficacy : The efficacy of a new pharmaceutical form may be evaluated by :

- the tumor growth inhibitor (T/C)

The T/C value in percent is an indication of antitumor effectiveness :

$$T/C\ (\%) = \frac{\text{Median tumor weight of the Treated}}{\text{Median tumor weight of the Control}} \times 100$$

According to NCI standards, a T/C < 42 % is the minimal level to declare activity. A T/C < 10 % is considered to indicate high antitumor activity and is the level used by NCI to justify further development (Decision Network-2 level, DN-2).

The T/C value can be converted to an arbitrary activity rating according to the NCI criteria :

| NCI activity | | % T/C |
|---|---|---|
| Highly active | ++ | ≤ 10 |
| Active | + | 11-42 |
| Inactive | - | > 42 |

- and quantified by the log cell kill which is determined

$$\log_{10} \text{ cells killed} = \text{T-C (days)} / 3.32 \times T_d$$

in which T-C represents the time in days for the tumours of the treated group (T) and the tumors of the non-treated group (C) to reach a predetermined value (750 mg for example) [T.H. CORBETT et al., Cancer, 40, 2660-2680 (1977) ; F.M. SCHABEL et al., Cancer Drug Development, Part B. Methods in Cancer Research, 17, 3-51, New York, Academic Press Inc. (1979)]. A product is considered to be active if $\log_{10}$ cells killed is greater than or equal to 0.7. A product is considered to be very active if logo cells killed is greater than 2.8.

Results :

| Agent | Route volume | Dosage in mg/kg/dose | Schedule in days | Total dose in mg/kg | Drug death (days of death) | Average body weight change in % per mouse post last treatment (day) | Median tumor weight in mg on day 12 (range) | T/C in % day 12 | Time for median tumor to reach 750 mg in days | T-C in days | log cell kill | Comments |
|---|---|---|---|---|---|---|---|---|---|---|---|---|
| Product of formula Ia | p.o. | 20.6 | 3 to 7 | 103.0 | 5/5 (7,2d8,10,11) | - | - | - | - | - | - | Toxic |
| | 0.2 ml | 12.8 | | 64.0 | 0/5 | -17.9 (10) | 0 (0-0) | 0 | 25.9 | 13.8 | 3.2 | HNTD higly active |
| | | 8.4 | | 42.0 | 0/5 | -11.0 (9) | 0 (0-268) | 0 | 18.2 | 6.1 | 1.4 | Active |
| | | 5.5 | | 27.5 | 0/5 | +0.2(8 | 271 (107-432) | 38 | 14.4 | 2.3 | 0.5 | Inactive |
| Control | | | | | | | 704 (312-864) | | 12.1 | | | |

Tumor doubling time = 1.3 days 10 mice in the control group. Mice average weight (product of formula Ia) = 20.39 g

Abbreviation used : HNTD = highest nontoxic dose

Comments : Product of formula Ia was evaluated against early stage mammary adenocarcinoma MA17/A using a daily schedule for 5 days. At the highest nontoxic dose 12.8 mg/kg/administration (total dose 64 mg/kg), product Ia was found highly active in this tumor model with a 0 % T/C and a 3.2 log cell kill total.

## Claims

**1.** Use of a compound of formula (I) for preparing a medecine for treating abnormal cell proliferation, said use comprising administration by oral route to a mammal of an effective amount of a compound of formula (I) or a pharmaceutically acceptable salt or solvate.

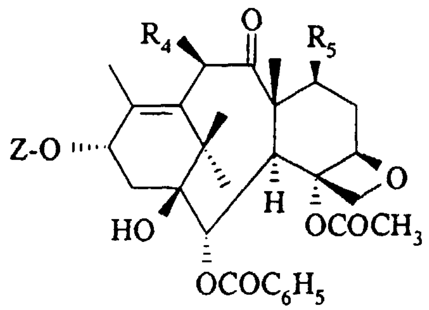

(I)

in which:

Z represents a hydrogen atom or a radical of general formula :

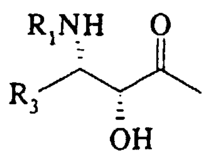

(II)

in which :
$R_1$ represents
a benzoyl radical optionally substituted with one or more identical or different atoms or radicals chosen from halogen atoms and alkyl radicals containing 1 to 4 carbon atoms, alkoxy radicals containing 1 to 4 carbon atoms or trifluoromethyl radicals,
a thenoyl or furoyl radical or
a radical $R_2$-O-CO- in which $R_2$ represents :

an alkyl radical containing 1 to 8 carbon atoms,
an alkenyl radical containing 2 to 8 carbon atoms,
an alkynyl radical containing 3 to 8 carbon atoms,
a cycloalkyl radical containing 3 to 6 carbon atoms,
a cycloalkenyl radical containing 4 to 6 carbon atoms or
a bicycloalkyl radical containing 7 to 10 carbon atoms,

these radicals being optionally substituted with one or more substituents chosen from halogen atoms and hydroxyl radicals, alkoxy radicals containing I to 4 carbon atoms, dialkylamino radicals in which each alkyl portion contains 1 to 4 carbon atoms, piperidino or morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, phenyl radicals (optionally substituted with one or more atoms or radicals chosen from halogen atoms and alkyl radicals containing 1 to 4 carbon atoms or alkoxy radicals containing 1 to 4 carbon atoms), cyano or carboxyl radicals or alkoxycarbonyl radicals in which the alkyl portion contains 1 to 4 carbon atoms,

- a phenyl or α- or β-naphthyl radical optionally substituted with one or more atoms or radicals chosen from halogen atoms and alkyl radicals containing 1 to 4 carbon atoms or alkoxy radicals containing 1 to 4 carbon atoms, or
- a 5-membered aromatic heterocyclic radical preferably chosen from furyl and thienyl radicals,

- or a saturated heterocyclic radical containing 4 to 6 carbon atoms, optionally substituted with one or more alkyl radicals containing 1 to 4 carbon atoms,

$R_3$ represents
an unbranched or branched alkyl radical containing 1 to 8 carbon atoms,
an unbranched or branched alkenyl radical containing 2 to 8 carbon atoms,
an unbranched or branched alkynyl radical containing 2 to 8 carbon atoms,
a cycloalkyl radical containing 3 to 6 carbon atoms,
a phenyl or α- or β-naphthyl radical optionally substituted with one or more atoms or radicals chosen from halogen atoms and alkyl, alkenyl, alkynyl, aryl, aralkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro and trifluoromethyl radicals,
or a 5-membered aromatic heterocycle containing one or more identical or different hetero atoms chosen from nitrogen, oxygen and sulphur atoms and optionally substituted with one or more identical or different substituents chosen from halogen atoms and alkyl, aryl, amino, alkylamino, dialkylamino, alkoxycarbonylamino, acyl, arylcarbonyl, cyano, carboxyl, carbamoyl, alkylcarbamoyl, dialkylcarbamoyl or alkoxycarbonyl radicals,
on the understanding that, in the substituents of the phenyl, α- or β-naphthyl and aromatic heterocyclic radicals, the alkyl radicals and the alkyl portions of the other radicals contain 1 to 4 carbon atoms, and that the alkenyl and alkynyl radicals contain 2 to 8 carbon atoms, and that the aryl radicals are phenyl or I- or 9-naphthyl radicals,
$R_4$ represents
an alkoxy radical containing 1 to 6 carbon atoms in an unbranched or branched chain,
an alkenyloxy radical containing 3 to 6 carbon atoms in an unbranched or branched chain,
an alkynyloxy radical containing 3 to 6 carbon atoms in an unbranched or branched chain,
a cycloalkyloxy radical containing 3 to 6 carbon atoms or
a cycloalkenyloxy radical containing 4 to 6 carbon atoms,
these radicals being optionally substituted with one or more halogen atoms or with an alkoxy radical containing 1 to 4 carbon atoms, an alkylthio radical containing 1 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur or nitrogen atoms, optionally substituted with an alkyl radical containing 1 to 4 carbon atoms or a phenyl radical or a phenylalkyl radical in which the alkyl portion contains 1 to 4 carbon atoms,
$R_5$ represents
an alkoxy radical containing 1 to 6 carbon atoms in an unbranched or branched chain,
an alkenyloxy radical containing 3 to 6 carbon atoms,
an alkynyloxy radical containing 3 to 6 carbon atoms,
a cycloalkyloxy radical containing 3 to 6 carbon atoms or
a cycloalkenyloxy radical containing 3 to 6 carbon atoms,
these radicals being optionally substituted with one or more halogen atoms or with an alkoxy radical containing 1 to 4 carbon atoms, an alkylthio radical containing 2 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur or nitrogen atoms, optionally substituted with an alkyl radical containing 1 to 4 carbon atoms or a phenyl radical or a phenylalkyl radical in which the alkyl portion contains 1 to 4 carbon atoms.

**2.** Use of compound according to claim 1 wherein Z represents a hydrogen atom or a radical of general formula (II) in which $R_1$ represents a benzoyl radical or a radical $R_2$-O-CO- in which $R_2$ represents a tert-butyl radical and $R_3$ represents an alkyl radical containing 1 to 6 carbon atoms, an alkenyl radical containing 2 to 6 carbon atoms, a cycloalkyl radical containing 3 to 6 carbon atoms, a phenyl radical optionally substituted with one or more identical or different atoms or radicals chosen from halogen atoms and alkyl, alkoxy, dialkylamino, acylamino, alkoxycarbonylamino or trifluoromethyl radical, or a 2- or 3-furyl, 2- or 3-thienyl or 2-, 4- or 5-thiazolyl radical, and $R_4$ and $R_5$, which may be identical or different, each represent an unbranched or branched alkyloxy radical containing 1 to 6 carbon atoms.

**3.** Use of a compound according to claim 2 wherein Z represents a hydrogen atom or a radical of general formula (II) in which $R_1$ represents a benzoyl radical or a radical $R_2$-O-CO- in which $R_2$ represents a tert-butyl radical and $R_3$ represents an isobutyl, isobutenyl, butenyl, cyclohexyl, phenyl, 2-furyl, 3-furyl, 2-thienyl, 3-thienyl, 2-thiazolyl, 4-thiazolyl or 5-thiazolyl radical, and $R_4$ and $R_5$, which may be identical or different, each represent a methoxy, ethoxy or propoxy radical.

**4.** Use of 4α-acetoxy-2α-benzoyloxy-5β,20-epoxy-1β-hydroxy-7β,10β-dimethoxy-9-oxo-11-taxen-13α-yl (2R,3S)-3-tert-butoxycarbonylamino-2-hydroxy-3-phenyl-propionate. for preparing a medecine for administration by oral route.

**5.** Oral solid dosage form containing a compound of claims 1 to 4 and an inert excipient, a binder and a disintegrating agent.

**6.** Oral liquid dosage form containing a compound of claims 1 to 4 and an inert diluent, solubilizing agent and emulsifier.

European Patent Office

# EUROPEAN SEARCH REPORT

Application Number
EP 99 40 0072

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,X | WO 96 30355 A (RHONE POULENC RORER SA) 3 October 1996<br>* abstract *<br>* examples *<br>* page 39, line 24 - page 40, line 29; claims *<br>--- | 1-6 | A61K31/335 |
| A | WO 97 32869 A (RHONE POULENC RORER SA ;BOUCHARD HERVE (FR); BOURZAT JEAN DOMINIQU) 12 September 1997<br>* abstract *<br>* examples *<br>* page 24, line 16 - page 25, line 21; claims *<br>--- | 1-6 | |
| A | EP 0 639 577 A (SQUIBB BRISTOL MYERS CO) 22 February 1995<br>* abstract *<br>* page 3, line 28 - page 4, line 15 *<br>* page 24, line 13 - line 35; claims 23-38 *<br>----- | 1-6 | TECHNICAL FIELDS SEARCHED (Int.Cl.6)<br>A61K |

The present search report has been drawn up for all claims

| Place of search | Date of completion of the search | Examiner |
|---|---|---|
| THE HAGUE | 4 June 1999 | Hoff, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 99 40 0072

This annex lists the patent family members relating to the patent documents cited in the above-mentioned European search report.
The members are as contained in the European Patent Office EDP file on
The European Patent Office is in no way liable for these particulars which are merely given for the purpose of information.

04-06-1999

| Patent document cited in search report | Publication date | Patent family member(s) | Publication date |
|---|---|---|---|
| WO 9630355 A | 03-10-1996 | FR 2732340 A | 04-10-1996 |
| | | FR 2742754 A | 27-06-1997 |
| | | AU 5278096 A | 16-10-1996 |
| | | AU 703278 B | 25-03-1999 |
| | | AU 5278196 A | 16-10-1996 |
| | | BG 101917 A | 30-10-1998 |
| | | BG 101918 A | 30-10-1998 |
| | | BR 9607929 A | 02-06-1998 |
| | | BR 9607930 A | 02-06-1998 |
| | | CA 2214319 A | 03-10-1996 |
| | | CA 2214321 A | 03-10-1996 |
| | | CZ 9703030 A | 14-01-1998 |
| | | CZ 9703032 A | 14-01-1998 |
| | | EP 0817779 A | 14-01-1998 |
| | | EP 0817780 A | 14-01-1998 |
| | | WO 9630356 A | 03-10-1996 |
| | | JP 11500141 T | 06-01-1999 |
| | | JP 11502531 T | 02-03-1999 |
| | | NO 973922 A | 26-08-1997 |
| | | NO 973923 A | 26-08-1997 |
| | | NZ 304900 A | 24-09-1998 |
| | | NZ 304901 A | 23-12-1998 |
| | | PL 322465 A | 02-02-1998 |
| | | PL 322499 A | 02-02-1998 |
| | | SK 130197 A | 08-04-1998 |
| | | SK 130297 A | 04-03-1998 |
| | | US 5847170 A | 08-12-1998 |
| | | US 5889043 A | 30-03-1999 |
| | | ZA 9602399 A | 01-10-1996 |
| WO 9732869 A | 12-09-1997 | FR 2745814 A | 12-09-1997 |
| | | AU 2030297 A | 22-09-1997 |
| | | CA 2248195 A | 12-09-1997 |
| | | CZ 9802834 A | 13-01-1999 |
| | | EP 0885208 A | 23-12-1998 |
| | | NO 983472 A | 28-07-1998 |
| | | PL 328691 A | 15-02-1999 |
| EP 0639577 A | 22-02-1995 | AU 660570 B | 29-06-1995 |
| | | AU 5260993 A | 07-07-1994 |
| | | AU 694941 B | 06-08-1998 |
| | | AU 7026794 A | 02-03-1995 |
| | | AU 9135698 A | 14-01-1999 |
| | | CA 2111527 A | 25-06-1994 |
| | | CA 2129288 A | 18-02-1995 |
| | | CN 1093369 A,B | 12-10-1994 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 99 40 0072

This annex lists the patent family members relating to the patent documents cited in the above-mentioned European search report.
The members are as contained in the European Patent Office EDP file on
The European Patent Office is in no way liable for these particulars which are merely given for the purpose of information.

04-06-1999

| Patent document cited in search report | Publication date | Patent family member(s) | Publication date |
|---|---|---|---|
| EP 0639577 A | | CN 1111637 A | 15-11-1995 |
| | | CN 1174838 A | 04-03-1998 |
| | | CZ 9401947 A | 16-08-1995 |
| | | CZ 9302855 A | 15-12-1994 |
| | | EP 0604910 A | 06-07-1994 |
| | | FI 935821 A | 25-06-1994 |
| | | FI 943749 A | 18-02-1995 |
| | | HU 65547 A | 28-06-1994 |
| | | HU 67742 A | 28-04-1995 |
| | | JP 7149779 A | 13-06-1995 |
| | | JP 7002885 A | 06-01-1995 |
| | | MX 9308012 A | 31-08-1994 |
| | | NO 934614 A | 27-06-1994 |
| | | NO 943002 A | 20-02-1995 |
| | | NZ 250550 A | 24-02-1995 |
| | | NZ 264240 A | 24-10-1997 |
| | | PL 301610 A | 27-06-1994 |
| | | PL 304649 A | 20-02-1995 |
| | | US 5646176 A | 08-07-1997 |
| | | ZA 9406180 A | 16-02-1995 |

EPO FORM P0459

For more details about this annex : see Official Journal of the European Patent Office, No. 12/82